# EUROPÄISCHE PATENTSCHRIFT

(11) **EP 2 417 084 B1**
(45) Veröffentlichungstag und Bekanntmachung des Hinweises auf die Patenterteilung: **26.11.2014**
(21) Anmeldenummer: 10711677.4
(22) Anmeldetag: 30.03.2010
(51) Int. Cl.: C01B 3/26, C01B 3/30, C01B 3/50, H01M 8/06, H01M 8/10, C07C 2/76

(54) **VERFAHREN ZUR UMSETZUNG VON ERDGAS ZU AROMATEN MIT ELEKTROCHEMISCHER ABTRENNUNG VON WASSERSTOFF UNTER ERZEUGUNG VON ELEKTRISCHEM STROM UND WASSERSTOFF**
METHOD FOR REACTING NATURAL GAS TO AROMATICS WHILE ELECTROCHEMICALLY REMOVING HYDROGEN AND WHILE GENERATING ELECTRIC POWER AND PRODUCING HYDROGEN
PROCÉDÉ DE TRANSFORMATION DE GAZ NATUREL EN HYDROCARBURES AROMATIQUES AVEC SÉPARATION ÉLECTROCHIMIQUE D'HYDROGÈNE, AVEC PRODUCTION CONCOMITANTE DE COURANT ÉLECTRIQUE ET D'HYDROGÈNE

(30) Priorität: 06.04.2009 EP 09157397
(43) Veröffentlichungstag der Anmeldung: 15.02.2012
(73) Patentinhaber: BASF SE, 67056 Ludwigshafen (DE)
(72) Erfinder: COELHO TSOU, Joana, 69120 Heidelberg (DE); PANCHENKO, Alexander, 67063 Ludwigshafen (DE); WENTINK, Annebart, Engbert, 68161 Mannheim (DE); AHRENS, Sebastian, 67059 Ludwigshafen (DE); HEIDEMANN, Thomas, 68519 Viernheim (DE)
(86) Internationale Anmeldenummer: PCT/EP2010/054155
(87) Internationale Veröffentlichungsnummer: WO 2010/115768

(56) Entgegenhaltungen:
- WO-A1-03/084905
- SOLYMOSI F ET AL: "Conversion of ethane into benzene on Mo2C/ZSM-5 catalyst" APPLIED CATALYSIS A: GENERAL, ELSEVIER SCIENCE, AMSTERDAM, NL LNKD- DOI:10.1016/S0926-860X(97)00260-3, Bd. 166, Nr. 1, 2. Januar 1998 (1998-01-02), Seiten 225-235, XP023613553 ISSN: 0926-860X [gefunden am 1998-01-02]

## Beschreibung

Die vorliegende Erfindung betrifft ein Verfahren zur Umsetzung von aliphatischen Kohlenwasserstoffen mit 1 bis 4 C-Atomen zu aromatischen Kohlenwasserstoffen in Gegenwart eines Katalysators unter nicht-oxidativen Bedingungen, wobei mindestens ein Teil des bei der Umsetzung entstandenen Wasserstoffs mittels einer Membran-Elektroden-Assembly elektrochemisch abgetrennt wird. Dabei wird auf der Retentatseite der Membran mindestens ein Teil des Wasserstoffs zu Protonen oxidiert. Die Protonen werden nach Durchqueren der Membran auf der Permeatseite zum einen Teil unter Anlegen einer Spannung zu Wasserstoff reduziert und zum anderen Teil unter Erzeugung elektrischen Stroms mit Sauerstoff zu Wasser umgesetzt. Der Sauerstoff stammt aus einem Sauerstoff enthaltenden Strom O, der mit der Permeatseite der Membran in Kontakt gebracht wird.

Aromatische Kohlenwasserstoffe wie Benzol, Toluol, Ethylbenzol, Styrol, Xylol und Naphthalin stellen bedeutende Zwischenprodukte in der chemischen Industrie dar, deren Bedarf nach wie vor steigt. In der Regel werden sie durch katalytische Reformierung aus Naphtha gewonnen, das seinerseits aus Erdöl erhalten wird. Neuere Untersuchungen zeigen, dass die weltweiten Erdölvorräte im Vergleich zu den Erdgasvorräten stärker begrenzt sind. Daher ist die Herstellung von aromatischen Kohlenwasserstoffen aus Edukten, die aus Erdgas gewonnen werden können, eine inzwischen auch wirtschaftlich interessante Alternative. Die Hauptkomponente von Erdgas stellt üblicherweise Methan dar.

Eine mögliche Reaktionsroute zur Gewinnung von Aromaten aus Aliphaten stellt die nichtoxidative Dehydroaromatisierung (DHAM) dar. Die Umsetzung erfolgt hierbei unter nicht-oxidativen Bedingungen, insbesondere unter Ausschluss von Sauerstoff. Bei der DHAM findet eine Dehydrierung und Cyclisierung der Aliphaten zu den entsprechenden Aromaten unter Freisetzung von Wasserstoff statt. Dabei entstehen aus 6 mol Methan 1 mol Benzol und 9 mol Wasserstoff.

Thermodynamische Betrachtungen zeigen, dass die Umsetzung durch die Lage des Gleichgewichtes limitiert wird (D. Wang, J. H. Lunsford und M. P. Rosynek, "Characterization of a Mo/ZSM-5 catalyst for the conversion of methane to benzene", Journal of Catalysis 169, 347-358 (1997)). Rechnungen unter Berücksichtigung der Komponenten Methan, Benzol, Naphthalin und Wasserstoff ergeben, dass die Gleichgewichtsumsätze für die isotherme Umsetzung von Methan zu Benzol (und Naphthalin) mit steigendem Druck und fallender Temperatur abnehmen, beispielsweise liegt der Gleichgewichtsumsatz bei 1 bar und 750 °C bei etwa 17 %.

Um das bei der Reaktion nicht umgesetzte Methan effizient zu nutzen, das heißt es erneut zur DHAM einzusetzen, sollte ein Großteil des in Reaktionsaustrag enthaltenen H₂ entfernt werden, da sonst durch den H₂ das Reaktionsgleichgewicht ungünstig in Richtung Methan verschoben wird und somit die Ausbeute an aromatischen Kohlenwasserstoffen niedriger ausfällt.

Ein Verfahren zur DHAM von Kohlenwasserstoffen, insbesondere von Erdgas, mit Abtrennung des H₂ sowie der aromatischen Kohlenwasserstoffe aus dem Produktgas und Rückführung des restlichen Produktgases in die Reaktionszone bzw. die erneute Umsetzung des Produktgases nach Abtrennung des Wasserstoffs und ohne vorherige Abtrennung der aromatischen Kohlenwasserstoffe in einer weiteren Reaktionsstufe wird in der US 7,019,184 B2 beschrieben. Als Methoden zur Abtrennung des H₂ werden wasserstoffselektive Membranen und Druckwechseladsorption genannt. Der abgetrennte Wasserstoff kann zur Energieerzeugung eingesetzt werden, beispielsweise in einer Verbrennungskammer oder in einer Brennstoffzelle.

Bei der Wasserstoffabtrennung mittels einer selektiv wasserstoffdurchlässigen Membran wandert der Wasserstoff als H₂-Molekül durch die Membran. Die Diffusionsgeschwindigkeit hängt dabei vom Partialdruckunterschied des Wasserstoffs zwischen Retentat- und Permiat-Seite der Membran ab. Dieser kann prinzipiell durch drei verschiedene Methoden beeinflusst werden: 1) Kompression des Feedgases, wodurch sich der Partialdruck erhöht, 2) Erzeugen eines Vakuums auf der Permeatseite oder 3) Verwenden eines Sweep-Gases auf der Permeatseite, das den Partialdruck des Wasserstoffs erniedrigt. Diese Methoden sind entweder mechanisch anspruchsvoll (Optionen 1) und 2)) oder erfordern die Trennung des Sweep-Gases vom Wasserstoff. Zudem müssen die entsprechenden Vorrichtungen zum Verdichten und Expandieren des Gasgemisches vorhanden sein. Aus kinetischen Gründen bleibt stets ein gewisser Anteil des Wasserstoffs im Retentat zurück. Beispielsweise enthält das Permeat einer H₂/CH₄-Mischung, das mittels einer wasserstoffdurchlässigen Polymermembran gewonnen wird, üblicherweise auf 10 Moleküle H₂ 1 Molekül CH₄. Bei einer Pd-Membran, die ab etwa 200°C selektiv wasserstoffdurchlässig wird und ihre optimale Trenneigenschaft bei 400°C bis 500°C erreicht, enthält das Permeat üblicherweise 1 Molekül CH₄ auf 200 Moleküle H₂.

Bei der Druckwechsel-Adsorption wird ein Adsorbens zyklisch in einer ersten Phase mit dem wasserstoffhaltigen Strom beaufschlagt, wobei alle Komponenten außer Wasserstoff durch Adsorption zurückgehalten werden. In einer zweiten Phase werden diese Komponenten durch erniedrigten Druck wieder desorbiert. Es handelt sich hierbei um ein technisch sehr aufwändiges Verfahren, bei dem Adsorbentien eingesetzt werden müssen und ein Wasserstoff enthaltender Abfallstrom entsteht, dessen Wasserstoffanteil mehr als 40 % betragen kann, siehe Ullmann's' Encyclopedia of Industrial Chemistry, "Membranes: Gas Separation-Applications", D.B. Strooky, Elah Strategies, S. 6, Chesterfield, Missouri, USA, 2005 Wiley-VCH Verlag, Weinheim.

Neben der Druckwechsel-Adsorption und dem Einsatz von selektiv wasserstoffdurchlässigen Membranen ist die Verwendung einer so genannten "Cold Box" ein übliches Verfahren zur Abtrennung von Wasserstoff aus Gasmischungen.

Bei der Wasserstoffabtrennung (WO-A-03/084905) mittels einer Cold Box wird das Gasgemisch unter Drücken von 30 bis 50 bar auf etwa -150°C bis -190°C abgekühlt. Die Erzeugung dieser tiefen Temperaturen ist kostspielig. Soll das dabei von Wasserstoff befreite Gemisch erneut in einer Reaktion eingesetzt werden, muss es auch wieder auf die entsprechende Reaktionstemperatur erwärmt werden, beispielsweise auf 600 bis 1000°C für die Dehydroaromatisierung.

Die Abtrennung von Wasserstoff aus einer Mischung aus Wasserstoff und Methan wird von B. Ibeh et al. (International Journal of Hydrogen Energy 32 (2007) Seiten 908 - 914) beschrieben. Ihr Ausgangspunkt war, die Eignung von Erdgas als Trägergas für den Transport von Wasserstoff durch die bereits vorhandene Infrastruktur für den Erdgastransport zu untersuchen, wobei der Wasserstoff nach dem gemeinsamen Transport mit dem Erdgas von diesem wieder abgetrennt werden muss. B. Ibeh et al. verwendeten zur Abtrennung von Wasserstoff aus Wasserstoff-Methan-Mischungen eine Brennstoffzelle mit einer einzelnen Protonen-Austausch-Membran und Pt oder Pt/Ru-Anodenelektrokatalysatoren. Der Brennstoffzelle wurden Wasserstoff-Methan-Mischungen bei Atmosphärendruck und Temperaturen zwischen 20 bis 70°C zugeführt.

Aufgabe der vorliegenden Erfindung ist, ein Verfahren zur Gewinnung von aromatischen Kohlenwasserstoffen aus aliphatischen Kohlenwasserstoffen mit 1 bis 4 Kohlenstoffatomen bereitzustellen, das die von den Verfahren aus dem Stand der Technik bekannten Nachteile nicht aufweist. Die eingesetzten aliphatischen Kohlenwasserstoffe sollen ebenso wie die bei der Umsetzung anfallenden Nebenprodukte effizient genutzt werden. Das Verfahren soll eine möglichst günstige Energiebilanz und einen möglichst geringen apparativen Aufwand aufweisen.

Die Aufgabe wird erfindungsgemäß durch das Verfahren zur Umsetzung von aliphatischen Kohlenwasserstoffen mit 1 bis 4 C-Atomen zu aromatischen Kohlenwasserstoffen umfassend die Schritte:
a) Umsetzung eines Eduktstroms E, der mindestens einen aliphatischen Kohlenwasserstoff mit 1 bis 4 C-Atomen enthält, in Gegenwart eines Katalysators unter nicht-oxidativen Bedingungen zu einem Produktstrom P enthaltend aromatische Kohlenwasserstoffe und Wasserstoff, und
b) elektrochemische Abtrennung mindestens eines Teils des bei der Umsetzung entstandenen Wasserstoffs aus dem Produktstrom P mittels einer gasdichten Membran-Elektroden-Assembly, die mindestens eine selektiv protonenleitende Membran und auf jeder Seite der Membran mindestens einen Elektrodenkatalysator aufweist, wobei auf der Retentatseite der Membran mindestens ein Teil des Wasserstoffs an dem Anodenkatalysator zu Protonen oxidiert wird und die Protonen nach Durchqueren der Membran auf der Permeatseite an dem Kathodenkatalysator teilweise gemäß
   b1) unter Anlegen einer Spannung zu Wasserstoff reduziert werden und teilweise gemäß
   b2) unter Erzeugung elektrischen Stroms mit Sauerstoff zu Wasser umgesetzt werden, wobei der Sauerstoff aus einem Sauerstoff enthaltenden Strom O stammt, der mit der Permeatseite der Membran in Kontakt gebracht wird,
gelöst.

Ein Vorteil des erfindungsgemäßen Verfahrens ist die elektrochemische Abtrennung des gebildeten Wasserstoffs aus dem Produktstrom P. Die Triebkraft der elektrochemischen Wasserstoffabtrennung ist der Potentialunterschied zwischen den beiden Seiten der Membran. Da die Abtrennung nicht, wie bei den üblich verwendeten wasserstoffselektiven Membranen, vom Partialdruckunterschied des Wasserstoffs zwischen den beiden Seiten der Membran abhängig ist, kann die Wasserstoffabtrennung bei sehr viel niedrigeren Drücken und Druckunterschieden durchgeführt werden, wobei vorzugsweise auf einen von außen aufgeprägten Druckunterschied völlig verzichtet wird und insbesondere auf Permeat- und Retentatseite der gleiche Druck herrscht. Damit verringert sich die mechanische Beanspruchung der Membran deutlich, was unter anderem ihre Langzeitstabilität erhöht sowie die Auswahl an für die Membran in Frage kommenden Materialien vergrößert.

Dies bietet weiterhin die Möglichkeit, die Abtrennung des Wasserstoffs bei niedrigeren Drücken durchzuführen als bei herkömmlichen Membranen und besitzt den Vorteil, dass das gesamte Verfahren, das heißt die DHAM sowie die Aufarbeitung des Produktstroms P, bei ähnlichen Drücken durchgeführt werden können. Es kann also auf aufwändige Vorrichtungen zum Verdichten und Expandieren der Gasströme verzichtet werden.

Die elektrochemische Abtrennung kann im Vergleich zur Abtrennung des Wasserstoffs mittels Cold Box bei hohen Temperaturen ausgeführt werden. Die Temperaturen für die elektrochemische Abtrennung des Wasserstoffs liegen üblicherweise bei Raumtemperatur oder höher. Die bei der Umsetzung entstandenen aromatischen Kohlenwasserstoffe werden üblicherweise nach Abkühlen des Produktstroms auf Temperaturen unterhalb des Siedepunktes des Benzols ausgewaschen und mittels eines Gasflüssigkeitsabscheiders abgetrennt. Da die elektrochemische Wasserstoffabtrennung auch oberhalb der hierbei erforderlichen Temperaturen durchgeführt werden kann, muss der Produktstrom P nicht stärker abgekühlt werden, als es für die Abtrennung der aromatischen Kohlenwasserstoffe nötig ist. Im Vergleich zur Cold-box werden Abkühlschritte und die dazu benötigten Vorrichtungen eingespart. Beim Rückführen des nicht umgesetzte C₁-C₄-Aliphate enthaltenden Produktstroms P in die Umsetzungszone nach Entfernen des Wasserstoffs und der aromatischen Kohlenwasserstoffe, muss der rückgeführte Produktstrom P deutlich weniger stark aufgeheizt werden als bei Abtrennung des Wasserstoffs mittels Cold Box. Dies spart Energie ein.

Pd-Membranen sind zur Abtrennung des Wasserstoffs im vorliegenden Verfahren weniger gut geeignet, da sie erst ab etwa 200°C beginnen, wasserstoffdurchlässig zu werden, so dass bei diesen Temperaturen nur sehr niedrige Flussraten durch die Membranen erreicht werden. Bei der optimalen Betriebstemperatur von etwa 400°C bis 500°C können an den Pd-Membranen jedoch unerwünschte Nebenreaktionen der im zu trennenden Produktstrom P enthaltenden Aliphaten stattfinden, die zu einer relativ schnellen Verkokung der Pd-Membranen führen und zu einem entsprechend hohem Regenerierungsbedarf.

Die elektrochemische Abtrennung des Wasserstoffs ist im Vergleich zur Abtrennung mittels herkömmlicher wasserstoffselektiver Membranen deutlich effektiver, bei gleicher Trennleistung kann daher die erforderliche Membranfläche verkleinert werden oder bei gleich bleibender Membranfläche deutlich mehr Wasserstoff abgetrennt werden, so dass der im rückgeführten Produktstrom P verbleibende Wasserstoffgehalt deutlich geringer ist. Insgesamt ist das erfindungsgemäße Verfahren daher mit weniger apparativem Aufwand verbunden.

Die Teilstufe b2) weist den besonderen Vorteil auf, die elektrochemische Abtrennung des gebildeten Wasserstoffs aus dem Produktstrom P mit der gleichzeitigen Gewinnung von elektrischem Strom zu kombinieren.

Der Wasserstoff wird dabei nicht, wie aus dem Stand der Technik bekannt, zunächst abgetrennt und anschließend als Wasserstoff einem Strom erzeugenden Verfahren wie einer externen Brennstoffzelle oder Gasturbine zugeführt, sondern die Stromerzeugung erfolgt schon bei der Abtrennung. Im Vergleich zu den aus dem Stand der Technik bekannten Verfahren wird, je nach Blickwinkel, eine Trennvorrichtung oder eine Einheit zur Energieerzeugung aus dem entstandenen Wasserstoff und die damit einhergehenden Energie- und Substanzverluste eingespart.

Ganz besonders vorteilhaft an dem erfindungsgemäßen Verfahren ist die Kombination aus den Teilschritten b1) und b2), da der in Teilschritt b2) erzeugte Strom direkt zur Reduktion der Wasserstoffprotonen in Teilschritt b1) eingesetzt werden kann. Wie viel Wasserstoff jeweils nach den Teilschritten b1) und b2) abgetrennt wird, lässt sich nach Wunsch einstellen, je nachdem, ob mehr Wasserstoff oder Strom erzeugt werden soll.

Das erfindungsgemäße Verfahren stellt somit eine wirtschaftliche Nutzung der eingesetzten Edukte unter gleichzeitiger flexibler Produktion von wertvollen aromatischen Kohlenwasserstoffen, Wasserstoff und elektrischer Energie bereit.

Im Folgenden wird die Erfindung ausführlich beschrieben.

Die Umsetzung des Eduktstroms E zu einem Produktstrom P findet in Gegenwart eines Katalysators unter nicht-oxidativen Bedingungen statt.

Nicht-oxidativ gemäß der vorliegenden Erfindung bedeutet in Bezug auf die DHAM, dass die Konzentration von Oxidationsmitteln wie Sauerstoff oder Stickoxiden im Eduktstrom E unterhalb von 5 Gew.-%, bevorzugt unterhalb von 1 Gew.-%, besonders bevorzugt unterhalb von 0,1 Gew.-% liegt. Ganz besonders bevorzugt ist der Eduktstrom E frei von Sauerstoff. Ebenfall besonders bevorzugt ist eine Konzentration an Oxidationsmitteln im Eduktstrom E, die gleich groß oder geringer ist als die Konzentration an Oxidationsmitteln in der Quelle, aus der die C₁-C₄-Aliphaten stammen.

Erfindungsgemäß enthält der Eduktstrom E mindestens einen Aliphaten mit 1 bis 4 Kohlenstoffatomen. Zu diesen Aliphaten gehören beispielsweise Methan, Ethan, Propan, n-Butan, i-Butan, Ethen, Propen, 1- und 2-Buten, Isobuten. In einer Ausführungsform der Erfindung enthält der Eduktstrom E mindestens 50 mol-%, bevorzugt mindestens 60 mol-%, besonders bevorzugt mindestens 70 mol-%, außerordentlich bevorzugt mindestens 80 mol-%, insbesondere mindestens 90 mol-% C₁-C₄-Aliphaten.

Unter den Aliphaten werden besonders bevorzugt die gesättigten Alkane verwendet, Eduktstrom E enthält dann bevorzugt mindestens 50 mol-%, bevorzugt mindestens 60 mol-%, besonders bevorzugt mindestens 70 mol-% außerordentlich bevorzugt mindestens 80 mol-%, insbesondere mindestens 90 mol-% Alkane mit 1 bis 4 C-Atomen.

Unter den Alkanen sind Methan und Ethan bevorzugt, insbesondere Methan. Gemäß dieser Ausführungsform der vorliegenden Erfindung enthält der Eduktstrom E bevorzugt mindestens 50 mol-%, bevorzugt mindestens 60 mol-%, besonders bevorzugt mindestens 70 mol-% außerordentlich bevorzugt mindestens 80 mol-%, insbesondere mindestens 90 mol-% Methan.

Bevorzugt wird als Quelle der C₁-C₄-Aliphaten Erdgas eingesetzt. Die typische Zusammensetzung von Erdgas sieht folgendermaßen aus: 75 bis 99 mol-% Methan, 0,01 bis 15 mol-% Ethan, 0,01 bis 10 mol-% Propan, bis zu 6 mol-% Butan, bis zu 30 mol-% Kohlendioxid, bis zu 30 mol-% Schwefelwasserstoff, bis zu 15 mol-% Stickstoff und bis zu 5 mol-% Helium. Das Erdgas kann vor dem Einsatz in dem erfindungsgemäßen Verfahren nach dem Fachmann bekannten Methoden gereinigt und angereichert werden. Zur Reinigung gehört beispielsweise die Entfernung von gegebenenfalls im Erdgas vorhandenen Schwefelwasserstoff oder Kohlendioxid und weiterer, im anschließenden Verfahren unerwünschter Verbindungen.

Die in dem Eduktstrom E enthaltenen C₁-C₄-Aliphaten können auch aus anderen Quellen stammen, beispielsweise bei der Erdölraffination angefallen sein. Die C₁-C₄-Aliphaten können auch regenerativ (z.B. Biogas) oder synthetisch (z.B. Fischer-Tropsch-Synthese) hergestellt worden sein.

Falls als C₁-C₄-Aliphaten-Quelle Biogas verwendet wird, kann der Eduktstrom E zusätzlich noch Ammoniak, Spuren von niederen Alkoholen und weitere, für Biogas typische Beimischungen enthalten.

In einer weiteren Ausführungsform des erfindungsgemäßen Verfahrens kann als Eduktstrom E LPG (Liquid Petroleum Gas) eingesetzt werden. Gemäß einer weiteren Ausführungsform des erfindungsgemäßen Verfahrens kann als Eduktstrom E LNG (Liquified Natural Gas) eingesetzt werden.

Dem Eduktstrom E kann zusätzlich Wasserstoff, Wasserdampf, Kohlenmonoxid, Kohlendioxid, Stickstoff sowie ein oder mehrere Edelgase beigemischt werden.

In Schritt a) des erfindungsgemäßen Verfahrens findet die Umsetzung des Eduktstroms E unter nicht-oxidativen Bedingungen in Gegenwart eines Katalysators zu einem aromatische Kohlenwasserstoffe enthaltenden Produktstrom P statt. Bei dieser Umsetzung handelt es sich um eine Dehydroaromatisierung, d.h. die in dem Eduktstrom E enthaltenen C₁-C₄-Aliphaten reagieren unter Dehydrierung und Cyclisierung zu den entsprechenden Aromaten, wobei Wasserstoff freigesetzt wird. Erfindungsgemäß wird die DHAM in Gegenwart geeigneter Katalysatoren durchgeführt. Generell können alle Katalysatoren, die die DHAM katalysieren, in Schritt a) des erfindungsgemäßen Verfahrens eingesetzt werden. Üblicherweise enthalten die DHAM-Katalysatoren einen porösen Träger und mindestens ein darauf aufgebrachtes Metall. Der Träger enthält üblicherweise eine kristalline oder amorphe anorganische Verbindung.

Erfindungsgemäß enthält der Katalysator bevorzugt mindestens ein Metallosilikat als Träger. Bevorzugt werden als Träger Aluminiumsilikate eingesetzt. Ganz besonders bevorzugt werden erfindungsgemäß als Träger Zeolithe verwendet. Bei Zeolithen handelt es sich um Aluminiumsilikate, die bei ihrer Herstellung üblicherweise in der Natriumform anfallen. In der Na-Form wird die wegen des Austausches von 4-wertigen Si-Atomen gegen 3-wertige Al-Atomen im Kristallgitter vorhandene überschüssige negative Ladung durch Na-Ionen ausgeglichen. Statt allein Natrium kann der Zeolith zum Ladungsausgleich auch weitere Alkali- und/oder Erdalkaliionen enthalten. Erfindungsgemäß bevorzugt weist der in den Katalysatoren enthaltene mindestens eine Zeolith eine Struktur auf, die aus den Strukturtypen Pentasil und MWW ausgewählt ist und besonders bevorzugt aus den Strukturtypen MFI, MEL, Mischstrukturen aus MFI und MEL und MWW ausgewählt ist. Ganz besonders bevorzugt wird ein Zeolith des Typs ZSM-5 oder MCM-22 eingesetzt. Die Bezeichnungen der Strukturtypen der Zeolithe entsprechen den Angaben in W. M. Meier, D. H. Olson und Ch. Baerlocher, "Atlas of Zeolithe Structure Types", Elsevier, 3. Auflage, Amsterdam 2001. Die Synthese der Zeolithe ist dem Fachmann bekannt und kann beispielsweise ausgehend von Alkalialuminat, Alkalisilikat und amorphem SiO₂ unter hydrothermalen Bedingungen durchgeführt werden. Hierbei kann über organische Templat-Moleküle, über die Temperatur und weitere experimentelle Parameter die Art der gebildeten Kanalsysteme im Zeolithen gesteuert werden.

Die Zeolithe können neben Al weitere Elemente wie Ga, B, Fe oder In enthalten.

Vorzugsweise werden die bevorzugt als Träger verwendeten Zeolithe in der H-Form oder der Ammoniumform, in denen die Zeolithe auch kommerziell erhältlich sind, eingesetzt.

Bei der Überführung von der Na-Form in die H-Form werden die im Zeolithen enthaltenden Alkali- und/oder Erdalkaliionen gegen Protonen ausgetauscht. Ein übliches und gemäß der vorliegenden Erfindung bevorzugtes Verfahren zum Überführen der Katalysatoren in die H-Form ist ein zweistufiger Prozess, bei dem die Alkali- und/oder Erdalkaliionen zunächst gegen Ammoniumionen ausgetauscht werden. Beim Erhitzen des Zeoliths auf etwa 400 bis 500°C zersetzt sich das Ammoniumion in flüchtigen Ammoniak und in das im Zeolithen verbleibende Proton.

Dazu wird der Zeolith mit einer NH₄-haltigen Mischung behandelt. Als NH₄-haltige Komponente der NH₄-haltigen Mischung wird ein Ammoniumsalz, ausgewählt aus der Gruppe Ammoniumchlorid, Ammoniumcarbonat, Ammoniumhydrogencarbonat, Ammoniumnitrat, Ammoniumphosphat, Ammoniumacetat, Ammoniumhydrogenphosphat, Ammoniumdihydrogenphosphat, Ammoniumsulfat und Ammoniumhydrogensulfat eingesetzt. Bevorzugt wird als NH₄-haltige Komponente Ammoniumnitrat verwendet.

Die Behandlung des Zeolithen mit der NH₄-haltigen Mischung erfolgt nach den bekannten, zum Ammoniumaustausch von Zeolithen geeigneten Methoden. Dazu zählt beispielsweise das Tränken, Tauchen oder Besprühen des Zeolithen mit einer Ammoniumsalzlösung, wobei die Lösung im Allgemeinen im Überschuss angewendet wird. Als Lösungsmittel werden vorzugsweise Wasser und/oder Alkohole verwendet. Die Mischung enthält üblicherweise 1 bis 20 Gew.-% der eingesetzten NH₄-Komponente. Die Behandlung mit der NH₄-haltigen Mischung wird üblicherweise über einen Zeitraum von mehreren Stunden und bei erhöhten Temperaturen durchgeführt. Nach dem Einwirken der NH₄-haltigen Mischung auf den Zeolithen kann überschüssige Mischung entfernt und der Zeolith gewaschen werden. Anschließend wird der Zeolith bei 40 bis 150 °C für mehrere Stunden, üblicherweise 4 bis 20 Stunden getrocknet. Daran schließt sich die Kalzinierung des Zeolithen bei Temperaturen von 300 bis 700 °C, bevorzugt von 350 bis 650 °C und besonders bevorzugt von 500 bis 600 °C an. Die Dauer der Kalzinierung beträgt üblicherweise 2 bis 24 Stunden, bevorzugt 3 bis 10 Stunden, besonders bevorzugt 4 bis 6 Stunden.

In einer bevorzugten Ausführungsform der vorliegenden Erfindung werden als Träger Zeolithe, die erneut mit einer NH₄-haltigen Mischung behandelt und anschließend getrocknet wurden, eingesetzt. Die erneute Behandlung der Zeolithe mit der NH₄-haltigen Mischung erfolgt gemäß der vorstehenden Beschreibung.

Kommerziell erhältliche Zeolithe in der H-Form haben üblicherweise bereits einen ersten Ammoniumaustausch durch Behandeln mit einer NH₄-haltigen Mischung mit anschließendem Trocknen und Kalzinieren durchlaufen. Deshalb können erfindungsgemäß kommerziell erworbene, in der H-Form vorliegende Zeolithe als Träger a) eingesetzt werden, bevorzugt werden sie jedoch einer erneuten Behandlung mit einer NH₄-haltigen Mischungen unterzogen und gegebenenfalls kalziniert.

Üblicherweise enthält der DHAM-Katalysator mindestens ein Metall. Üblicherweise wird das Metall ausgewählt aus den Gruppen 3 bis 12 des Periodensystems der Elemente (IU-PAC). Erfindungsgemäß bevorzugt enthält der DHAM-Katalysator mindestens ein Metall ausgewählt aus den Übergangsmetallen der Nebengruppen 5 bis 11. Besonders bevorzugt enthält der DHAM-Katalysator mindestens ein Metall ausgewählt aus der Gruppe Mo, W, Mn, Tc, Re, Fe, Ru, Os, Co, Rh, Ir, Ni, Pd, Pt, Cu, Cr, Nb, Ta, Ag und Au. Insbesondere enthält der DHAM-Katalysator mindestens ein Metall ausgewählt aus der Gruppe Mo, W, Re, Fe, Ru, Os, Co, Rh, Ir, Ni, Pd, Pt, Cu. Ganz besonders bevorzugt enthält der DHAM-Katalysator mindestens ein Metall ausgewählt aus der Gruppe Mo, W und Re.

Erfindungsgemäß ebenfalls bevorzugt enthält der DHAM-Katalysator mindestens ein Metall als Aktivkomponente und mindestens ein weiteres Metall als Dotierung. Die Aktivkomponente wird erfindungsgemäß ausgewählt aus Mo, W, Re, Ru, Os, Rh, Ir, Pd, Pt. Die Dotierung wird erfindungsgemäß ausgewählt aus der Gruppe Cr, Mn, Fe, Co, Nb, Ta, Ni, Cu, V, Zn, Zr und Ga, bevorzugt aus der Gruppe Fe, Co, Nb, Ta, Ni, Cu und Cr. Erfindungsgemäß kann der DHAM-Katalysator mehr als ein Metall als Aktivkomponente und mehr als ein Metall als Dotierung enthalten. Diese werden jeweils aus den für die Aktivkomponente und die Dotierung angegebenen Metallen ausgewählt. Bevorzugt enthält der Katalysator ein Metall als Aktivkomponente und ein oder zwei Metalle als Dotierung.

Das mindestens eine Metall wird erfindungsgemäß nasschemisch oder trockenchemisch gemäß den dem Fachmann bekannten Methoden auf den Träger aufgebracht.

Nasschemisch werden die Metalle in Form wässriger, organischer oder organischwässriger Lösungen ihrer Salze oder Komplexe durch Imprägnieren des Trägers mit der entsprechenden Lösung aufgebracht. Als Lösungsmittel kann auch überkritisches CO₂ dienen. Die Imprägnierung kann nach der incipient-wetness-Methode erfolgen, bei der das poröse Volumen des Trägers durch in etwa gleiches Volumen an Imprägnierlösung aufgefüllt wird und man - gegebenenfalls nach einer Reifung - den Träger trocknet. Man kann auch mit einem Überschuss an Lösung arbeiten, wobei das Volumen dieser Lösung größer ist als das poröse Volumen des Trägers. Hierbei wird der Träger mit der Imprägnierlösung gemischt und ausreichend lange gerührt. Weiterhin ist es möglich, den Träger mit einer Lösung der entsprechenden Metallverbindung zu besprühen. Es sind auch andere, dem Fachmann bekannte Herstellmethoden wie Ausfällen der Metallverbindungen auf dem Träger, Aufsprühen einer Metallverbindung enthaltenden Lösung, Soltränkung etc. möglich. Nach dem Aufbringen des mindestens einen Metalls auf den Träger wird der Katalysator bei etwa 80 bis 130°C üblicherweise 4 bis 20 Stunden im Vakuum oder an Luft getrocknet.

Erfindungsgemäß kann das mindestens eine Metall auch auf trockenchemischem Wege aufgebracht werden, beispielsweise indem die bei höheren Temperaturen gasförmigen Metallcarbonyle wie Mo(CO)₆, W(CO)₆ und Re₂(CO)₁₀ aus der Gasphase auf dem Träger abgeschieden werden. Das Abscheiden der Metallcarbonylverbindung wird im Anschluss an das Kalzinieren des Trägers durchgeführt. Es kann auch in Form eines feinen Pulvers, beispielsweise als Carbid, mit dem Träger vermischt werden.

Erfindungsgemäß enthält der Katalysator 0,1 bis 20 Gew.-%, bevorzugt 0,2 bis 15 Gew.-%, besonders bevorzugt 0,5 bis 10 Gew.-%, jeweils bezogen auf das Gesamtgewicht des Katalysators des mindestens einen Metalls. Der Katalysator kann nur ein Metall enthalten, er kann auch ein Gemisch aus zwei, drei oder mehr Metallen enthalten. Die Metalle können nasschemisch gemeinsam in einer Lösung aufgebracht werden oder in verschiedenen Lösungen nacheinander mit Trocknungsschritten zwischen den einzelnen Auftragungen. Die Metalle können auch gemischt aufgebracht werden, d.h. ein Teil nasschemisch und ein anderer Teil trockenchemisch. Zwischen den Auftragungen der Metallverbindungen kann nach Bedarf entsprechend der vorstehenden Beschreibung kalziniert werden. Erfindungsgemäß kann der Katalysator mindestens ein Metall aus der Gruppe der Aktivkomponente in Verbindung mit mindestens einem Metall ausgewählt aus der Gruppe der Dotierung enthalten. In diesem Fall liegt die Konzentration der Aktivkomponente bei 0,1 bis 20 Gew.-%, bevorzugt 0,2 bis 15 Gew.-%, besonders bevorzugt bei 0,5 bis 10 Gew.-%, jeweils bezogen auf das Gesamtgewicht des Katalysators. Die Dotierung liegt in diesem Fall im Katalysator erfindungsgemäß in einer Konzentration von mindestens 0,1 Gew.-%, bevorzugt mindestens 0,2 Gew.-%, ganz besonders bevorzugt mindestens 0,5 Gew.-%, bezogen auf das Gesamtgewicht des Katalysators vor.

Gemäß einer weiteren bevorzugten Ausführungsform der vorliegenden Erfindung wird der Katalysator mit einem Bindemittel vermischt. Als Bindemittel eignen sich die üblichen, dem Fachmann bekannten Bindemittel wie Aluminiumoxid- und/oder Si-haltige Bindemittel. Besonders bevorzugt sind dabei Si-haltige Bindemittel; insbesondere eignen sich Tetraalkoxysilane, Polysiloxane und kolloidale SiO₂-Sole.

Erfindungsgemäß erfolgt nach Zugabe des Bindemittels ein Formgebungsschritt, in dem die Katalysatormasse gemäß den dem Fachmann bekannten Verfahren zu Formkörpern verarbeitet werden. Als formgebende Verfahren sind dabei beispielsweise Versprühen einer den Träger a) bzw. die Katalysatormasse enthaltenden Suspension, Sprühtrocknung, Tablettieren, Verpressen im feuchten oder trockenen Zustand und Extrudieren zu nennen. Zwei oder mehrere dieser Verfahren können auch kombiniert werden. Für das Verformen können Hilfsmittel wie Porenbildner und Anteigungsmittel oder auch andere, dem Fachmann bekannte Zusatzstoffe eingesetzt werden. Mögliche Anteigungsmittel sind solche Verbindungen, die zur Verbesserung der Misch-, Knet- und Fließeigenschaften führen. Vorzugsweise sind dies im Rahmen der vorliegenden Erfindung organische, insbesondere hydrophile Polymere wie beispielsweise Cellulose, Cellulosederivate wie Methylcellulose, Stärke wie Kartoffelstärke, Tapetenkleister, Acrylate, Polyacrylate, Polymethacrylate, Polyvinylalkohole, Polyvinalpyrrolidon, Polyisobutylen, Polytetrahydrofuran, Polyglykolether, Fettsäureverbindungen, Wachsemulsionen, Wasser oder Mischungen aus zwei oder mehr dieser Verbindungen. Als Porenbildner sind im Rahmen der vorliegenden Erfindung beispielsweise in Wasser oder wässrigen Lösungsmittelgemischen dispergier-, suspendier- oder emulgierbare Verbindungen wie Polyalkylenoxide, Polystyrol, Polyacrylate, Polymethacrylate, Polyolefine, Polyamide, Polyester, Kohlenhydrate, Cellulose, Cellulosederivate wie beispielsweise Methylcellulose, Zuckernaturfasern, Pulp, Graphit oder Mischungen aus zwei oder mehr dieser Verbindungen zu nennen. Porenbildner und/oder Anteigungsmittel werden nach der Verformung bevorzugt durch mindestens einen geeigneten Trocknungs- und/oder Kalzinierungsschritt aus dem erhaltenen Formkörper entfernt. Die dazu erforderlichen Bedingungen können analog zu den vorstehend für Kalzinierung beschriebenen Parametern gewählt werden und sind dem Fachmann bekannt. Insbesondere für den Einsatz als Wirbelschichtkatalysatoren werden die Katalysatorformkörper mittels Sprühtrocknung hergestellt.

Die Geometrie der erfindungsgemäß erhältlichen Katalysatoren kann beispielsweise kugelförmig (hohl oder voll), zylindrisch (hohl oder voll), ring-, sattel-, stern-, bienenwaben- oder tablettenförmig sein. Weiterhin kommen Extrudate beispielsweise in Strang-, Trilob-, Quatrolob, Stern- oder Hohlzylinderform in Frage. Weiterhin kann die zu formende Katalysatormasse extrudiert, kalziniert und die so erhaltenen Extrudate gebrochen und zu Split oder Pulver verarbeitet werden. Der Split kann in verschiedene Siebfraktionen getrennt werden.

Gemäß einer bevorzugten Ausführungsform der Erfindung wird der Katalysator als Formkörper oder Split eingesetzt.

Gemäß einer weiteren bevorzugten Ausführungsform wird der Katalysator als Pulver eingesetzt. Das Katalysatorpulver kann dabei Bindemittel enthalten, aber auch frei von Bindemittel vorliegen.

Wenn der erfindungsgemäße Katalysator ein Bindemittel enthält, liegt dies in einer Konzentration von 5 bis 80 Gew.-%, bezogen auf das Gesamtgewicht des Katalysators vor, bevorzugt von 10 bis 50 Gew.-%, besonders bevorzugt von 10 bis 30 Gew.-%.

Es kann von Vorteil sein, den zur Dehydroaromatisierung von C₁-C₄-Aliphaten verwendeten Katalysator vor der eigentlichen Reaktion zu aktivieren.

Diese Aktivierung kann mit einem C₁-C₄-Alkan, wie z.B. Ethan, Propan, Butan oder einem Gemisch hiervon, vorzugsweise Butan, erfolgen. Die Aktivierung wird bei einer Temperatur von 250 bis 850°C, vorzugsweise bei 350 bis 650°C, und einem Druck von 0,5 bis 5 bar, vorzugsweise bei 0,5 bis 2 bar, durchgeführt. Üblicherweise liegt die GHSV (Gas Hourly Space Velocity) bei der Aktivierung bei 100 bis 4000 h⁻¹, vorzugsweise bei 500 bis 2000 h⁻¹.

Es ist aber auch möglich eine Aktivierung durchzuführen, indem der Eduktstrom E das C₁-C₄-Alkan, oder ein Gemisch hiervon, per se schon enthält oder das C₁-C₄-Alkan, oder ein Gemisch hiervon, dem Eduktstrom E zugesetzt wird. Die Aktivierung wird bei einer Temperatur von 250 bis 650°C, vorzugsweise bei 350 bis 550°C, und einem Druck von 0,5 bis 5 bar, vorzugsweise bei 0,5 bis 2 bar, durchgeführt.

In einer weiteren Ausgestaltungsform ist es auch möglich zusätzlich zu dem C₁-C₄-Alkan noch Wasserstoff beizufügen.

Gemäß einer bevorzugten Ausführungsform der vorliegenden Erfindung wird der Katalysator mit einem H₂ enthaltenden Gasstrom aktiviert, der zusätzlich Inertgase wie N₂, He, Ne und Ar enthalten kann.

Erfindungsgemäß wird die Dehydroaromatisierung von C₁-C₄-Aliphaten in Gegenwart eines Katalysators bei Temperaturen von 400 bis 1000 °C, bevorzugt von 500 bis 900°C, besonders bevorzugt von 600 bis 800°C, insbesondere von 700 bis 800 °C, bei einem Druck von 0.5 bis 100 bar, bevorzugt bei 1 bis 30 bar, besonders bevorzugt bei 1 bis 10 bar, insbesondere 1 bis 5 bar, durchgeführt. Gemäß der vorliegenden Erfindung wird die Umsetzung bei einer GHSV (Gas Hourly Space Velocity) von 100 bis 10 000 h⁻¹, vorzugsweise von 200 bis 3000 h⁻¹ durchgeführt.

Die Dehydroaromatisierung von C₁-C₄-Aliphaten gemäß Schritt a) kann grundsätzlich in allen aus dem Stand der Technik bekannten Reaktortypen durchgeführt werden. Eine geeignete Reaktorform ist der Festbett-, Radialstrom-, Rohr- oder Rohrbündelreaktor. Bei diesen befindet sich der Katalysator als Festbett in einem Reaktionsrohr oder in einem Bündel von Reaktionsrohren. Ebenso können die Katalysatoren als Wirbelschicht, Wanderbett oder Fließbett in den entsprechenden, dafür geeigneten Reaktortypen eingesetzt werden und das erfindungsgemäße Verfahren mit den derart vorliegenden Katalysatoren durchgeführt werden.

Erfindungsgemäß kann der Katalysator unverdünnt oder mit Inertmaterial vermischt eingesetzt werden. Als Inertmaterial kann jedes Material dienen, das sich bei den in den Reaktionszonen herrschenden Reaktionsbedingungen inert verhält, d.h. nicht reagiert. Als Inertmaterial eignet sich besonders der undotierte, für den Katalysator eingesetzte Träger, aber auch inerte Zeolithe, Aluminiumoxid, Siliziumdioxid etc. Die Teilchengröße des Inertmaterials liegt im Bereich der Größe der Katalysatorteilchen.

Bevorzugt gemäß der vorliegenden Erfindung liegt der unverdünnte Katalysator oder mit Inertmaterial vermischte, als Fest-, Wander- oder Wirbelbett vor. Besonders bevorzugt liegt der Katalysator bzw. das Gemisch aus Katalysator und Inertmaterial als Wirbelschicht vor.

Der bei der DHAM eingesetzte Katalysator wird gemäß einer Ausführungsform der Erfindung regelmäßig regeneriert. Die Regenerierung kann gemäß den üblichen, dem Fachmann bekannten Verfahren durchgeführt werden. Erfindungsgemäß bevorzugt wird die Regenerierung unter reduzierenden Bedingungen mittels eines Wasserstoff enthaltenden Gasstroms durchgeführt.

Die Regenerierung wird bei Temperaturen von 600 °C bis 1000 °C und besonders bevorzugt von 700 °C bis 900 °C und Drücken von 1 bar bis 30 bar, bevorzugt von 1 bar bis 15 bar und besonders bevorzugt von 1 bis 10 bar durchgeführt.

Die C₁-C₄-Aliphaten werden erfindungsgemäß unter Freisetzung von H₂ zu Aromaten umgesetzt. Der Produktstrom P enthält daher mindestens einen aromatischen Kohlenwasserstoff ausgewählt aus der Gruppe Benzol, Toluol, Ethylbenzol, Styrol, Xylol und Naphthalin. Besonders bevorzugt enthält er Benzol und Toluol. Weiterhin enthält der Produktstrom nicht umgesetzte C₁-C₄-Aliphate, entstandenen Wasserstoff und die im Eduktstrom E enthaltenen Inertgase wie N₂, He, Ne, Ar, dem Eduktstrom E zugesetzte Stoffe wie H₂ sowie bereits im Eduktstrom E vorhandene Verunreinigungen.

In Schritt b) des erfindungsgemäßen Verfahrens wird mindestens ein Teil des im Produktstrom P enthaltenen Wasserstoffs elektrochemisch mittels einer gasdichten Membran-Elektroden-Assembly abgetrennt, wobei der abzutrennende Wasserstoff in Form von Protonen durch die Membran transportiert wird. Die Elektroden mit der dazwischen angeordneten Membran werden Membran-Elektroden-Assembly (MEA) genannt. Der Produktstrom P wird auf einer Seite der Membran entlanggeführt. Diese Seite wird im Folgenden Retentatseite genannt. Die andere Seite der Membran wird im Folgenden als Permeatseite bezeichnet. Auf dieser Seite wird der gemäß Teilschritt b1) entstandene Wasserstoff und das gemäß Teilschritt b2) entstandene Wasser abgeführt. Erfindungsgemäß weist die MEA mindestens eine selektiv protonenleitende Membran auf. Die Membran weist auf jeder Seite mindestens einen Elektrodenkatalysator auf, wobei im Rahmen dieser Beschreibung der auf der Retentatseite befindliche Elektrodenkatalysator als Anodenkatalysator, der auf der Permeatseite befindliche Elektrodenkatalysator als Kathodenkatalysator bezeichnet wird. Auf der Retentatseite wird der Wasserstoff am Anodenkatalysator zu Protonen oxidiert, diese durchqueren die Membran und werden auf der Permeatseite am Kathodenkatalysator teilweise zu Wasserstoff (b1)) reduziert und teilweise unter Erzeugung von elektrischer Energie mit Sauerstoff zu Wasser umgesetzt (b2)); in Teilschritt b2) wird dazu an der Permeatseite ein Sauerstoff enthaltender Strom O entlanggeführt und mit der Membran in Kontakt gebracht. Bei dem Teilschritt b1) muss für den Transport der Protonen durch die Membran elektrische Energie aufgewendet werden, die durch Anlegen einer Gleichspannung an die beiden Seiten der Membran mittels Elektroden zugeführt wird.

Um einen guten Kontakt der Membran mit dem auf der Retentatseite befindlichen Wasserstoff und einen guten Abtransport des abgetrennten Wasserstoffs bzw. des Wassers auf der Permeatseite zu gewährleisten, sind die Elektrodenschichten üblicherweise mit Gasverteilerschichten kontaktiert. Diese sind zum Beispiel Platten mit einer gitterartigen Oberflächenstruktur aus einem System feiner Kanäle oder Schichten aus porösem Material wie Vlies, Gewebe oder Papier. Die Gesamtheit von Gasverteilerschicht und Elektrodenschicht wird im Allgemeinen als Gasdiffusionselektrode (GDE) bezeichnet. Durch die Gasverteilerschicht wird der abzutrennende Wasserstoff auf der Retentatseite nahe an die Membran und den Anodenkatalysator geführt und auf der Permeatseite der Abtransport des gebildeten Wasserstoffs bzw. Wassers erleichtert.

Je nach Ausführungsform der Erfindung kann die Anode gleichzeitig auch als Anodenkatalysator und die Kathode gleichzeitig auch als Kathodenkatalysator dienen. Es können jedoch auch jeweils unterschiedliche Materialien für die Anode und den Anodenkatalysator bzw. die Kathode und den Kathodenkatalysator verwendet werden.

Zur Herstellung der Elektroden können die üblichen, dem Fachmann bekannten Materialien verwendet werden. Beispielsweise können die Elektroden Pt, Pd, Cu, Ni, Fe, Ru, Co, Cr, Fe, Mn, V, W, Wolframcarbid, Mo, Molybdäncarbid, Zr, Rh, Ag, Ir, Au, Re, Y, Nb, elektrisch leitende Formen von Kohlenstoff wie Ruß, Graphit und Nanoröhrchen sowie Legierungen und Mischungen der vorstehend genannten Elemente enthalten.

Die Anode und die Kathode können aus dem gleichen Material oder aus unterschiedlichen Materialien hergestellt sein. Der Anodenkatalysator und der Kathodenkatalysator können aus den gleichen oder aus unterschiedlichen Materialien ausgewählt sein. Besonders bevorzugt sind als Anoden/Kathoden-Kombination Pt/Pt, Pd/Pd, Pt/Pd, Pd/Pt , Ni/Ni, Fe/Fe und Ni/Pt.

Als Elektrodenkatalysatormaterial können die üblichen, dem Fachmann bekannten Verbindungen und Elemente verwendet werden, die die Dissoziation von molekularem Wasserstoff in atomaren Wasserstoff, die Oxidation von Wasserstoff zu Protonen sowie die Reduktion von Protonen zu Wasserstoff katalysieren können. Geeignet sind beispielsweise Pd, Pt, Cu, Ni, Fe, Ru, Co, Cr, Mn, V, W, Wolframcarbid, Mo, Molybdäncarbid, Zr, Rh, Ag, Ir, Au, Re, Y, Nb sowie Legierungen und Mischungen davon, bevorzugt sind erfindungsgemäß Pd und Pt. Die vorstehend als Elektrodenkatalysatormaterial genannten Elemente und Verbindungen können auch geträgert vorliegen, bevorzugt wird dabei Kohlenstoff als Träger eingesetzt.

Gemäß einer bevorzugten Ausführungsform der vorliegenden Erfindung werden bevorzugt Kohlenstoff als leitendes Material enthaltende Elektroden verwendet. Dabei wird der Kohlenstoff und ein Elektrodenkatalysator bevorzugt auf einem porösen Träger wie Vlies, Gewebe oder Papier aufgebracht. Der Kohlenstoff kann dabei mit dem Katalysator vermischt oder zunächst der Kohlenstoff und anschließend der Katalysator aufgebracht werden. Gemäß einer weiteren Ausführungsform der Erfindung wird das als Elektrode eingesetzte elektrisch leitende Material und der Elektrodenkatalysator direkt auf der Membran aufgebracht.

Die Membran ist vorzugsweise als Platte oder als Rohr ausgebildet, wobei die üblichen, aus dem Stand der Technik zur Auftrennung von Gasgemischen bekannten Membrananordnungen verwendet werden können beispielsweise Rohrbündel- oder Steckplattenmembran.

Die erfindungsgemäß eingesetzte MEA ist gasdicht, das heißt sie weist praktisch keine Porosität auf, durch die Gase in atomarer oder molekularer Form von einer Seite auf die andere Seite der MEA gelangen können, noch weist sie Mechanismen auf, durch die Gase unselektiv beispielsweise durch Adsorption, Lösung in der Membran, Diffusion und Desorption durch die MEA transportiert werden können.

Die Dichtigkeit der Membran-Elektroden-Assembly (MEA) kann durch eine gasdichte Membran, durch eine gasdichte Elektrode und/oder einen gasdichten Elektrodenkatalysator gewährleistet werden. So kann als gasdichte Elektrode zum Beispiel eine dünne metallische Folie verwendet werden, beispielsweise eine Pd-, Pd-Ag- oder Pd-Cu-Folie.

Die erfindungsgemäß eingesetzte Membran leitet selektiv Protonen, das heißt insbesondere, dass sie nicht elektronenleitend ist. Erfindungsgemäß können für die Membranen alle dem Fachmann bekannten Materialien eingesetzt werden, aus denen sich protonenleitende Membranen formen lassen. Hierzu gehören beispielsweise die von J. W. Phair und S. P. S. Badwal in Ionics (2006) 12, Seiten 103 - 115 aufgeführten Materialien. Auch selektiv protonenleitende Membranen, wie sie aus der Brennstoffzellentechnik bekannt sind, können erfindungsgemäß verwendet werden.

Beispielsweise können bestimmte Heteropolysäuren wie H₃Sb₃B₂O₁₄ • 10H20, H₂Ti₄O₉ • 12H₂O und HSbP₂O₈ • 10 H₂O; saure Zirkoniumsilikate, -phosphate und -phosphonate in Schichtstruktur wie K₂ZrSi₃O₉, K₂ZrSi₃O₉, alpha-Zr(HPO₄)₂ • nH₂O, gamma-Zr(PO₄)-(H₂PO₄) • 2H₂O, alpha- und gamma-Zr-Sulfophenylphosphonat oder Sulfoarylphosphonat; nicht geschichtete Oxidhydrate wie Antimonsäure (Sb₂O₅ • 2H₂O), V₂O₅ • nH₂O, ZrO₂ • nH₂O, SnO₂ • nH₂O und Ce(HPO₄)₂ • nH₂O eingesetzt werden. Weiterhin können Oxosäuren und Salze, die zum Beispiel Sulfat-, Selenat-, Phosphat-, Arsenat-, Nitrat-Gruppen usw. enthalten, verwendet werden. Besonders geeignet sind Oxoanionensysteme auf Basis von Phosphaten oder komplexen Heteropolysäuren wie Polyphosphatgläser, Aluminiumpolyphosphat, Ammoniumpolyphosphat und Polyphosphatzusammensetzungen wie NH₄PO₃/(NH₄)₂SiP₄O₁₃ und NH₄PO₃/TiP₂O₇. Weiterhin sind oxidische Materialien einsetzbar wie Brownmillerite, Fluorite und Phosphate mit Apatitstruktur, Pyrochlor-Mineralien und Perovskite.

Perovskite weisen die Grundformel AB₁₋ₓMₓO_{3-y} auf, wobei M ein trivalentes Seltenerdenelement ist, das zur Dotierung dient, und y den Sauerstoffmangel im Perovskitoxid-Gitter bezeichnet. A kann beispielsweise ausgewählt werden aus Mg, Ca, Sr und Ba. B kann unter anderem ausgewählt werden aus Ce, Zr und Ti. Für A, B und M können auch unabhängig voneinander verschiedene Elemente aus den jeweiligen Gruppen gewählt werden.

Weiterhin können strukturell modifizierte Gläser eingesetzt werden wie Chalcogenid-Gläser, PbO-SiO₂, BaO-SiO₂ und CaO-SiO₂.

Eine weitere Klasse Materialien, die sich für die Herstellung von gasdichten und selektiv protonenleitenden Membranen eignen, sind Polymermembranen. Geeignete Polymere sind sulfonierte Polyetheretherketone (S-PEEK), sulfonierte Polybenzoimidazole (S-PBI) und sulfonierte Fluorkohlenwasserstoffpolymere (NAFION^{®}). Weiterhin können perflurierte Polysulfonsäuren, Polymere auf Styrolbasis, Poly(arylenether), Polyimide und Polyphosphazene eingesetzt werden.

Erfindungsgemäß werden als Materialien für die selektiv protonenleitende Membran bevorzugt die vorstehend genannten Polymere und die auf Phosphaten basierenden Verbindungen eingesetzt. Ganz besonders bevorzugt werden Membranen aus Polybenzamidazolen eingesetzt, insbesondere MEAs, die auf Polybenzimidazol und Phosphorsäure basieren, wie sie beispielsweise unter dem Namen Celtec-P^{®} von der BASF SE vertrieben werden.

Bei Verwendung von Polymermembranen werden diese üblicherweise durch die Gegenwart von etwa 0,5 bis 50 Vol.-% Wasser auf mindestens einer Seite, besser jedoch auf beiden Seiten der Membran befeuchtet. Auf der Retentatseite kann die Konzentration durch Wasserzugabe zum Eduktstrom E, zum Produktstrom P, zur Reaktionszone oder bei Rückführung des von H₂ und Aromaten befreiten Produktstroms P, der bereits eine gewisse Menge Wasser aus seinem bereits erfolgten Durchlauf durch das Verfahren enthält, eingestellt. Auch auf der Permeatseite wird üblicherweise durch Zudosierung von Wasser für die erforderliche Wasserkonzentration gesorgt, dies ist bei dem Teilschritt b2) jedoch nicht notwendig.

Ebenfalls bevorzugt werden keramische Membranen eingesetzt.

Geeignete protonenleitende Keramiken sind beispielsweise in Solid State lonics 125, (1999), 271-278; Journal of Power Sources 180, (2008), 15-22; lonics 12, (2006), 103-115; Journal of Power Sources 179 (2008) 92-95; Journal of Power Sources 176 (2008) 122-127 und Electrochemistry Communications 10 (2008) 1005-1007 beschrieben.

Beispiele für protonenleitende Keramiken und Oxide sind SrCeO₃, BaCeO₃, Yb:SrCeO₃, Nd:BaCeO₃, Gd:BaCeO₃, Sm:BaCeO₃, BaCaNd0₉, Y:BaCeO₃, Y:BaZrCeO₃, Pr-dotiertes Y:BaCeO₃, Gd:BaCeO₃, BaCe_{0,9}Y_{0,1}O_{2,95} (BYC), SrCe_{0,95}Yb_{0,05}O_{3-α}, BaCe_{0,9}Nd0_{,10}O_{3-α}, CaZr_{0,96}In_{0,04}O_{3-α}, (α bezeichnet die Anzahl der Sauerstofffehlstellen pro Formeleinheit des Oxides vom Perowskittyp); Sr-dotiertes La₃P₃O₉, Sr-dotiertes LaPO₄, BaCe_{0,9}Y_{0,1}O_{3-α} (BCY), BaZr_{0,9}Y_{0,1}O_{3-α} (BZY), Ba₃Ca_{1,18}Nb_{1,82}O_{8,73} (BCN18), (La_{1,95}Ca_{0,05})Zr₂O_{7-α}, La₂Ce₂O₇, Eu₂Zr₂O₇, H₂S/(B₂S₃ oder Ga₂S₃)/GeS₂, SiS₂, As₂S₃ oder Csl; BaCe_{0,8}Gd_{0,2}O_{3-α} (BCGO); Gd-dotierte BaCeO₃ wie BaCe_{0,85}Y_{0,15}O_{3-α} (BCY15) und BaCe_{0,8}Sm_{0,2}O_{3-α}, xAl₂O₃ (1-x)SiO₂ (x=0,0-1,0), SnP₂O₇, Sn_{1-X}In_{X}P₂O₇ (x = 0,0 - 0,2).

Die Abtrennung des Wasserstoffs in Schritt b) des erfindungsgemäßen Verfahrens kann bei Temperaturen von 20 bis 1200°C, bevorzugt von 20 bis 800°C, besonders bevorzugt von 20 bis 500°C und ganz besonders bevorzugt bei 70 bis 250°C durchgeführt werden. Bei Verwendung von MEAs basierend auf Polybenzimidazol und Phosphoräsure wird die Abtrennung bevorzugt bei 130 bis 200°C vorgenommen.

Die Abtrennung des Wasserstoffs in Schritt b) des erfindungsgemäßen Verfahrens wird vorzugsweise bei Drücken von 0,5 bis 10 bar, bevorzugt von 1 bis 6 bar, besonders bevorzugt von 1 bis 4 bar vorgenommen. Gemäß einer bevorzugten Ausführungsform der Erfindung liegt der Druckunterschied zwischen der Retentat- und der Permeatseite der Membran unter 1 bar, bevorzugt unter 0,5 bar, besonders bevorzugt besteht kein Durckunterschied.

Die Abtrennung des Wasserstoffs in Schritt b) wird erfindungsgemäß bei Spannungen von 0,05 bis 2000 mV, bevorzugt von 100 bis 1500 mV, besonders bevorzugt von 100 bis 900 mV und ganz besonders bevorzugt von 100 bis 800 mV gegenüber einer RHE (Wasserstoff-Referenzelektrode) durchgeführt.

Der Sauerstoff enthaltende Strom, der in Teilschritt b2) eingesetzt wird, enthält erfindungsgemäß mindestens 15 mol-%, bevorzugt mindestens 20 mol-% Sauerstoff. In einer bevorzugten Ausführungsform wird Luft als Sauerstoff enthaltender Strom O eingesetzt oder mit Sauerstoff angereicherte Luft. Die Luft wird üblicherweise ungereinigt verwendet.

Die Flussrate des Stroms O wird so gewählt, dass die O₂-Menge stöchiometrisch das 1- bis 10-fache, bevorzugt das 1,2- bis 5-fache und besonders bevorzugt das 1,5- bis 2,5-fache der H₂-Menge beträgt.

Erfindungsgemäß wird in Schritt b) mindestens ein Teil des bei der DHAM entstandenen Wasserstoffs abgetrennt. Bevorzugt werden mindestens 30 %, besonders bevorzugt mindestens 50 %, besonders bevorzugt mindestens 70 % und ganz besonders bevorzugt mindestens 95 %, insbesondere mindestens 98 % abgetrennt.

Der auf der Permeatseite gemäß Teilschritt b1) erhaltene Wasserstoff enthält höchstens 5 mol-%, bevorzugt höchstens 2 mol-% und besonders bevorzugt höchstens 1 mol-% Aliphaten mit 1 bis 4 C-Atomen. Darüber hinaus kann der Wasserstoff je nach verwendeter selektiv Protonen leitender Membran bis zu 50 Vol.-%, bevorzugt bis zu 20 Vol.-%, besonders bevorzugt bis zu 5 Vol.-% Wasser enthalten. Die Gegenwart von Wasser ist bei einigen Membrantypen, zum Beispiel bei bestimmten Polymermembranen zur Befeuchtung der Membran erforderlich.

Die elektrochemische Abtrennung des Wasserstoffs gemäß Schritt b) findet erfindungsgemäß außerhalb der Reaktionszone, in der Schritt a) durchgeführt wird, statt.

Die Abtrennung der im Produktstrom P enthaltenen aromatischen Kohlenwasserstoffe erfolgt nach den dem Fachmann bekannten Verfahren.

Gemäß einer Ausführungsform werden die entstandenen aromatischen Kohlenwasserstoffe zwischen Schritt a) und b) aus dem Produktstrom P abgetrennt. Gemäß einer weiteren Ausführungsform der Erfindung werden die entstandenen aromatischen Kohlenwasserstoffe nach Schritt b) aus dem Produktstrom abgetrennt.

In einer besonders bevorzugten Ausführungsform der Erfindung wird der Produktstrom P nach Abtrennung der aromatischen Kohlenwasserstoffe und mindestens eines Teils des Wasserstoffs in das Verfahren zurückgeführt, der Produktstrom P wird entweder dem Eduktstrom E oder direkt in die Reaktionszone für die DHAM zurückgeführt. Dabei ist erfindungsgemäß bevorzugt, dass möglichst viel Wasserstoff vor der Rückführung abgetrennt wird, da Wasserstoff das Reaktionsgleichgewicht bei der DHAM auf die Seite der Edukte verschiebt. Der zurückgeführte Produktstrom P enthält vorzugsweise 0 bis 2 mol-%, bevorzugt 0 bis 1 mol-% Wasserstoff.

Die vorstehend beschriebenen Katalysatoren auf Basis von zweimal mit NH₃-Lösung behandelten Zeolithen haben auch ohne den im Stand der Technik üblichen Zusatz von Wasserstoff zum Eduktstrom eine lange Lebensdauer und sind daher besonders gut geeignet, um bei der Rückführung des Produktstroms P nach Abtrennung eines möglichst großen Teils des entstandenen Wasserstoffs als DHAM-Katalysatoren eingesetzt zu werden.

Der gemäß Teilschritt b1) abgetrennte Wasserstoff kann vor der Weiterverwendung noch getrocknet werden, dies wird insbesondere durchgeführt, wenn in Schritt b) Polymermembranen eingesetzt werden, die befeuchtet werden müssen.

Mindestens ein Teil des Wasserstoffs wird in Schritt b) erfindungsgemäß jeweils teilweise nach den Teilschritten b1) und b2) abgetrennt. Dies bedeutet, dass der eine Teil des Wasserstoffs als Wasserstoff und der andere Teil des Wasserstoffs unter Erzeugung von elektrischer Energie als Wasser abgetrennt wird. Wie viel des im Produktstrom P enthaltenen Wasserstoffs jeweils nach den Teilschritten b1) und b2) abgetrennt wird, kann vom Anwender nach Bedarf angepasst werden. Gemäß einer bevorzugten Ausführungsform der Erfindung wird mindestens soviel Wasserstoff gemäß b2) abgetrennt, dass der dabei erzeugte Strom ausreicht, um den Energiebedarf für die Wasserstoffabtrennung gemäß Teilschritt b1) zu decken, d.h., der für die Abtrennung des Wasserstoffs gemäß Teilschritt b1) benötigte Strom wird durch den Teilschritt b2) erzeugt.

Die Abtrennung des Wasserstoffs aus dem Produktstrom P wird vorzugsweise räumlich getrennt durchgeführt, da bei Gegenwart von Sauerstoff auf der Permeatseite üblicherweise die Protonen direkt zu Wasserstoff reagieren. Der Produktstrom P kann beispielsweise nacheinander zunächst an einer MEA entlang geleitet werden, die auf der Permeatseite mit einem Strom O in Kontakt steht, so dass ein Teil des Wasserstoffs als Wasser abgetrennt wird. Anschließend wird der Produktstrom P an einer MEA entlang geführt werden, an die eine Spannung angelegt ist, so dass der Wasserstoff als Wasserstoff abgetrennt wird. Die räumliche Trennung zwischen den beiden Teilschritten b1) und b2) kann auch darin bestehen, dass der Produktstrom P zwischen zwei beispielsweise sich gegenüberliegenden Membranen hindurchgeleitet wird, von denen eine auf der Permeatseite mit einem Strom O in Kontakt steht und an die andere eine Spannung angelegt wird.

### Beispiele:

In den Beispielen 1 und 2 wird die Abtrennung des Wasserstoffs gemäß Teilschritt b1) gezeigt, in Beispiel 3 die Abtrennung gemäß Teilschritt b2).

### Beispiel 1:

Es wurde eine Membran-Elektroden-Assembly mit einer aktiven Fläche von 5 cm² eingesetzt , die eine auf mit Phosphorsäure gefüllten Polybenzimidazol basierende Membran aufwies. Diese Membran ist unter der Bezeichnung Celtec P^{®} von der BASF Fuel Cell GmbH erhältlich. Für die Anode sowie die Kathode wurden jeweils Gasdiffusionselektroden verwendet, die unter der Bezeichnung ELAT^{®} ebenfalls bei der BASF Fuel Cell GmbH erhältlich sind. Sowohl die Anode als auch die Kathode enthielten jeweils 1 mg/cm² Platin. Die Experimente wurden bei 160°C Betriebstemperatur und bei Atmosphärendruck durchgeführt. Das Gasgemisch wurde für die Trennversuche vorgemischt und enthielt 11,40 mol-% Wasserstoff, 88,10 mol-% Methan, 5000 mol ppm Ethen, 100 mol ppm Benzol und 50 mol ppm Ethan. Bei unterschiedlichen, jeweils konstant gehaltenen Gasflüssen auf der Anodenseite wurde die Spannung variiert. Das auf der Permeatseite erhaltene Gasgemisch wurde gaschromatographisch analysiert und die Stromdichten gemessen. In den Tabellen 1 und 2 sind die erreichten Wasserstoffumsätze und Stromdichten gezeigt, wobei der Umsatz H₂ die abgetrennte Menge H₂ in % bezogen auf den im Anodengasstrom enthaltenen Wasserstoff bezeichnet.

**Tabelle 1:**

| Fluss [ml/min] | Umsatz H₂ [%] | | |
|---|---|---|---|
| | U = 200 mV | U = 400 mV | U = 600 mV |
| 100 | 82 | 88 | 87 |
| 200 | 60 | 79 | 75 |
| 300 | 50 | 69 | 70 |
| 500 | 33 | 50 | 52 |
| 1000 | 17 | 26 | 31 |

**Tabelle 2:**

| Fluss [ml/min] | Stromdichte [A/cm²] | | |
|---|---|---|---|
| | U = 200 mV | U = 400 mV | U = 600 mV |
| 100 | 0,50 | 0,52 | 0,50 |
| 200 | 0,86 | 0,80 | 0,88 |
| 300 | 0,74 | 0,98 | 0,98 |
| 500 | 0,82 | 1,18 | 1,28 |
| 1000 | 0,86 | 1,30 | 1,54 |

### Beispiel 2

Die Versuchsparameter und Versuchsvorrichtungen sind die gleichen wie in Beispiel 1, mit dem Unterschied, dass die Kathode anstelle von Pt 1 mg/cm² Pd enthielt. Die Ergebnisse sind in den Tabellen 3 und 4 dargestellt.

**Tabelle 3:**

| Fluss [ml/min] | Umsatz H₂ [%] | | |
|---|---|---|---|
| | U = 200 mV | U = 400 mV | U = 600 mV |
| 100 | 84 | 93 | 92 |
| 200 | 61 | 77 | 77 |
| 300 | 47 | 71 | 65 |
| 500 | 26 | 39 | 41 |
| 1000 | 8 | 14 | 18 |

**Tabelle 4:**

| Fluss [ml/min] | Stromdichte [A/cm²] | | |
|---|---|---|---|
| | U = 200 mV | U = 400 mV | U = 600 mV |
| 100 | 0,52 | 0,56 | 0,56 |
| 200 | 0,67 | 0,86 | 0,82 |
| 300 | 0,73 | 0,94 | 0,94 |
| 500 | 0,82 | 1,10 | 1,14 |
| 1000 | 0,84 | 1,20 | 1,30 |

### Beispiel 3:

Die Versuchsbedingungen waren die gleichen wie in Beispiel 1, jedoch wurde auf der Kathodenseite Luft und auf der Anodenseite das Gasgemisch mit jeweils gleichen Flussraten entlang geführt und keine Spannung angelegt. Bei unterschiedlichen, jeweils konstant gehaltenen Gasflüssen wurde das auf der Permeatseite erhaltene Gasgemisch gaschromatographisch analysiert und die Stromdichten gemessen. In Tabelle 5 sind die erreichten Wasserstoffumsätze und Stromdichten gezeigt, wobei der Umsatz H₂ die abgetrennte Menge H₂ in % bezogen auf den im Anodengasstrom enthaltenen Wasserstoff bezeichnet.

**Tabelle 5**

| Anodenfluss [ml/min] | Stromdichte [A/cm²] | Umsatz H₂ [%] |
|---|---|---|
| 100 | 0,24 | 33 |
| 200 | 0,26 | 20 |
| 300 | 0,27 | 14 |
| 500 | 0,28 | 6 |
| 1000 | 0,29 | 0,2 |

## Patentansprüche

1. Verfahren zur Umsetzung von aliphatischen Kohlenwasserstoffen mit 1 bis 4 C-Atomen zu aromatischen Kohlenwasserstoffen umfassend die Schritte:
a) Umsetzung eines Eduktstroms E, der mindestens einen aliphatischen Kohlenwasserstoff mit 1 bis 4 C-Atomen enthält, in Gegenwart eines Katalysators unter nicht-oxidativen Bedingungen zu einem Produktstrom P enthaltend aromatische Kohlenwasserstoffe und Wasserstoff, und
b) elektrochemische Abtrennung mindestens eines Teils des bei der Umsetzung entstandenen Wasserstoffs aus dem Produktstrom P mittels einer gasdichten Membran-Elektroden-Assembly, die mindestens eine selektiv protonenleitende Membran und auf jeder Seite der Membran mindestens einen Elektrodenkatalysator aufweist, wobei auf der Retentatseite der Membran mindestens ein Teil des Wasserstoffs an dem Anodenkatalysator zu Protonen oxidiert wird und die Protonen nach Durchqueren der Membran auf der Permeatseite an dem Kathodenkatalysator teilweise gemäß
b1) unter Anlegen einer Spannung zu Wasserstoff reduziert werden und teilweise gemäß
b2) unter Erzeugung elektrischen Stroms mit Sauerstoff zu Wasser umgesetzt werden, wobei der Sauerstoff aus einem Sauerstoff enthaltenden Strom O stammt, der mit der Permeatseite der Membran in Kontakt gebracht wird.

2. Verfahren gemäß Anspruch 1, **dadurch gekennzeichnet, dass** die entstandenen aromatischen Kohlenwasserstoffe aus dem Produktstrom P zwischen Schritt a) und b) oder nach Schritt b) abgetrennt werden.

3. Verfahren gemäß einem Anspruch 1 oder 2, **dadurch gekennzeichnet, dass** der Produktstrom P nach Abtrennung mindestens eines Teils des Wasserstoffs und der aromatischen Kohlenwasserstoffe in das Verfahren zurückgeführt wird.

4. Verfahren gemäß einem der Ansprüche 1 bis 3, **dadurch gekennzeichnet, dass** mindestens ein Teil des bei b2) erzeugten elektrischen Stroms in b1) eingesetzt wird.

5. Verfahren gemäß einem der Ansprüche 1 bis 4, **dadurch gekennzeichnet, dass** der zur Abtrennung des Wasserstoffs gemäß b1) benötigte Strom durch b2) erzeugt wird.

6. Verfahren gemäß einem der Ansprüche 1 bis 5, **dadurch gekennzeichnet, dass** Schritt b) bei Temperaturen von 20 bis 1200°C durchgeführt wird.

7. Verfahren gemäß einem der Ansprüche 1 bis 6, **dadurch gekennzeichnet, dass** der Sauerstoff enthaltende Strom O mindestens 15 mol-% Sauerstoff enthält.

8. Verfahren gemäß einem der Ansprüche 1 bis 7, **dadurch gekennzeichnet, dass** als Sauerstoff enthaltender Strom O Luft eingesetzt wird.

9. Verfahren gemäß einem der Ansprüche 1 bis 8, **dadurch gekennzeichnet, dass** Schritt b) bei Drücken von 0,5 bis 10 bar durchgeführt wird.

10. Verfahren gemäß einem der Ansprüche 1 bis 9, **dadurch gekennzeichnet, dass** in Schritt b) auf der Retentatseite und der Permeatseite der gleiche Druck herrscht.

11. Verfahren gemäß einem der Ansprüche 1 bis 10, **dadurch gekennzeichnet, dass** Schritt b1) bei Spannungen von 0,05 bis 2000 mV gegenüber einer WasserstoffReferenzelektrode durchgeführt wird.

12. Verfahren gemäß einem der Ansprüche 1 bis 11, **dadurch gekennzeichnet, dass** als selektiv protonenleitende Membran Membranen ausgewählt aus der Gruppe Polymermembranen und keramische Membranen eingesetzt werden.

13. Verfahren gemäß einem der Ansprüche 1 bis 12, **dadurch gekennzeichnet, dass** als Elektroden Gasdiffusionselektroden eingesetzt werden.

14. Verfahren gemäß einem der Ansprüche 1 bis 13, **dadurch gekennzeichnet, dass** der Eduktstrom E mindestens 50 mol-% Methan enthält.

15. Verfahren gemäß einem der Ansprüche 1 bis 14, **dadurch gekennzeichnet, dass** der Eduktstrom E aus Erdgas stammt.

## Claims

1. A process for converting aliphatic hydrocarbons having 1 to 4 carbon atoms to aromatic hydrocarbons, comprising the steps of:
a) converting a reactant stream E which comprises at least one aliphatic hydrocarbon having 1 to 4 carbon atoms in the presence of a catalyst under nonoxidative conditions to a product stream P comprising aromatic hydrocarbons and hydrogen, and
b) electrochemically removing at least some of the hydrogen formed in the conversion from the product stream P by means of a gas-tight membrane-electrode assembly which has at least one selectively proton-conducting membrane and, on each side of the membrane, at least one electrode catalyst, at least some of the hydrogen being oxidized to protons over the anode catalyst on the retentate side of the membrane, and the protons, after passing through the membrane, on the permeate side over the cathode catalyst, are partly, in
b1) reduced to hydrogen with application of a voltage, and partly, in
b2) reacted with oxygen to give water to generate electrical power, the oxygen originating from an oxygen-comprising stream 0 which is contacted with the permeate side of the membrane.

2. The process according to claim 1, wherein the aromatic hydrocarbons formed are removed from the product stream P between steps a) and b) or after step b).

3. The process according to claim 1 or 2, wherein the product stream P, after removal of at least some of the hydrogen and of the aromatic hydrocarbons, is recycled into the process.

4. The process according to any of claims 1 to 3, wherein at least a portion of the electrical power generated in b2) is used in b1).

5. The process according to any of claims 1 to 4, wherein the power required to remove the hydrogen in b1) is generated by b2).

6. The process according to any of claims 1 to 5, wherein step b) is performed at temperatures of 20 to 1200°C.

7. The process according to any of claims 1 to 6, wherein the oxygen-comprising stream 0 comprises at least 15 mol% of oxygen.

8. The process according to any of claims 1 to 7, wherein the oxygen-comprising stream 0 used is air.

9. The process according to any of claims 1 to 8, wherein step b) is performed at pressures of 0.5 to 10 bar.

10. The process according to any of claims 1 to 9, wherein the pressure in step b) is the same on the retentate side and the permeate side.

11. The process according to any of claims 1 to 10, wherein step b1) is performed at voltages of 0.05 to 2000 mV relative to a hydrogen reference electrode.

12. The process according to any of claims 1 to 11, wherein the selectively proton-conducting membranes used are membranes selected from the group of polymer membranes and ceramic membranes.

13. The process according to any of claims 1 to 12, wherein the electrodes used are gas diffusion electrodes.

14. The process according to any of claims 1 to 13, wherein the reactant stream E comprises at least 50 mol% of methane.

15. The process according to any of claims 1 to 14, wherein the reactant stream E originates from natural gas.

## Revendications

1. Procédé pour la conversion d'hydrocarbures aliphatiques ayant de 1 à 4 atomes de carbone en hydrocarbures aromatiques, comprenant les étapes :
a) conversion d'un courant de produit de départ E, qui contient au moins un hydrocarbure aliphatique ayant de 1 à 4 atomes de carbone, en présence d'un catalyseur dans des conditions non oxydantes, en un courant de produit P contenant des hydrocarbures aromatiques et de l'hydrogène, et
b) séparation électrochimique d'au moins une partie de l'hydrogène formé dans la réaction d'avec le courant de produit P au moyen d'un assemblage membrane-électrode imperméable aux gaz, qui comporte au moins une membrane laissant passer sélectivement les protons et de chaque côté de la membrane au moins un catalyseur sur électrode, du côté rétentat de la membrane au moins une partie de l'hydrogène étant oxydée en protons au niveau du catalyseur sur l'anode et les protons après avoir traversé la membrane du côté perméat, au niveau du catalyseur sur la cathode en partie selon
b1) avec application d'une tension, étant réduits en hydrogène et en partie selon
b2) avec génération de courant électrique, étant convertis en eau avec de l'oxygène, l'oxygène provenant d'un courant 0 contenant de l'oxygène, qui est mis en contact avec le côté perméat de la membrane.

2. Procédé selon la revendication 1, **caractérisé en ce qu'**entre les étapes a) et b) ou après l'étape b) on sépare du courant de produit P les hydrocarbures aromatiques produits.

3. Procédé selon l'une quelconque des revendications 1 et 2, **caractérisé en ce qu'**après séparation d'au moins une partie de l'hydrogène et des hydrocarbures aromatiques le courant de produit P est recyclé dans le procédé.

4. Procédé selon l'une quelconque des revendications 1 à 3, **caractérisé en ce qu'**au moins une partie du courant électrique engendré en b2) est utilisée en b1).

5. Procédé selon l'une quelconque des revendications 1 à 4, **caractérisé en ce que** le courant requis pour la séparation de l'hydrogène selon b1) est engendré par b2).

6. Procédé selon l'une quelconque des revendications 1 à 5, **caractérisé en ce qu'**on effectue l'étape b) à des températures de 20 à 1 200 °C.

7. Procédé selon l'une quelconque des revendications 1 à 6, **caractérisé en ce que** le courant 0 contenant de l'oxygène contient au moins 15 % en moles d'oxygène.

8. Procédé selon l'une quelconque des revendications 1 à 7, **caractérisé en ce qu'**en tant que courant 0 contenant de l'oxygène on utilise de l'air.

9. Procédé selon l'une quelconque des revendications 1 à 8, **caractérisé en ce qu'**on effectue l'étape b) sous des pressions de 0,5 à 10 bars.

10. Procédé selon l'une quelconque des revendications 1 à 9, caractérisé en ce dans l'étape b) la même pression règne du côté rétentat et du côté perméat.

11. Procédé selon l'une quelconque des revendications 1 à 10, **caractérisé en ce qu'**on effectue l'étape b) sous des tensions de 0,05 à 2 000 mV par rapport à une électrode à hydrogène de référence.

12. Procédé selon l'une quelconque des revendications 1 à 11, **caractérisé en ce qu'**on utilise comme membrane laissant passer sélectivement les protons des membranes choisies dans le groupe des membranes polymères et des membranes céramiques.

13. Procédé selon l'une quelconque des revendications 1 à 12, **caractérisé en ce qu'**on utilise comme électrodes des électrodes à diffusion de gaz.

14. Procédé selon l'une quelconque des revendications 1 à 13, **caractérisé en ce que** le courant de produit de départ E contient au moins 50 % en moles de méthane.

15. Procédé selon l'une quelconque des revendications 1 à 14, **caractérisé en ce que** le courant de produit de départ E provient de gaz naturel.
